# EUROPÄISCHE PATENTANMELDUNG

(11) **EP 1 357 101 A1**
(43) Veröffentlichungstag der Anmeldung: **29.10.2003**
(21) Anmeldenummer: 03008041.0
(22) Anmeldetag: 14.04.2003
(51) Int. Cl.: C07C 29/09, C07C 67/24, C07C 69/157, C07C 33/22

(54) **Verfahren zur Herstellung von Benzylalkoholen**

(30) Priorität: 24.04.2002 DE 10218243
(71) Anmelder: Bayer Aktiengesellschaft, 51368 Leverkusen (DE)
(72) Erfinder: Ooms, Pieter, Dr., 47800 Krefeld (DE); Schenke, Bernd-Ulrich, Dr., 46236 Bottrop (DE)

(57) **Zusammenfassung**

Benzylalkohole können durch Umsetzung von Dibenzylether mit Ameisensäure zu Ameisensäurebenzylestern in Gegenwart von Säuren als Katalysator und Fragmentierung der Ameisensäurebenzylester hergestellt werden.

## Beschreibung

Die vorliegende Erfindung betrifft ein Verfahren zur Herstellung von Benzylalkoholen durch Umsetzung von Dibenzylethern mit Ameisensäure zu Ameisensäurebenzylestern in Gegenwart von Säuren als Katalysator und anschließend eine Fragmentierung der Ameisensäurebenzylester.

Die Herstellung von Benzylalkohol durch Hydrolyse von Benzylchlorid in Gegenwart von Basen ist seit langem bekannt (Ullmann's encyclopedia of industrial chemistry, 5. Edition, Weinheim, 1985, S.1.). Nachteilig ist die Bildung von Salzen, die entsorgt werden müssen und somit die Wirtschaftlichkeit dieser Verfahren verringern.

In der EP-A 0 752 404 und der EP-A 0 781 748 wird die Herstellung von Benzylalkohol durch Umsetzung von Benzylchlorid mit Wasser ohne Salzanfall beschrieben.

Auch bei diesem Verfahren fällt Dibenzylether als Nebenprodukt an.

Es bestand also die Aufgabe, ausgehend von Dibenzylether ein Verfahren zur Herstellung von Benzylalkohol bereitzustellen, welches unter milden Reaktionsbedingungen durchführbar ist und zu guten Ausbeuten führt.

Es wurde ein Verfahren zur Herstellung von Benzylalkoholen der Formel 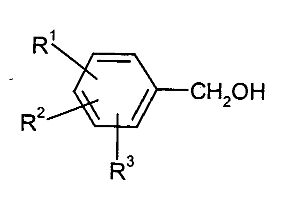 worin
R¹ bis R³ gleich oder verschieden sind und
für Wasserstoff, C₁-C₆-Alkyl, C₁-C₆-Alkoxy-, C₁-C₆-Halogenalkyl, C₁-C₆-Halogenalkoxy, CN, CO(C₁-C₆-Alkyl), NO₂ oder Halogen stehen,
gefunden, das dadurch gekennzeichnet ist, dass Dibenzylether der Formel 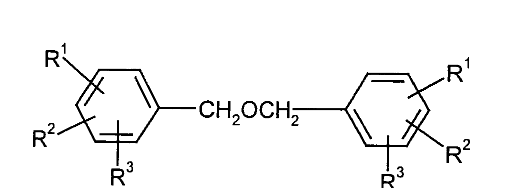 worin
R¹, R ² und R ³ die oben genannte Bedeutungen haben,
oder Gemische aus Dibenzylethern und Benzylalkoholen der Formel 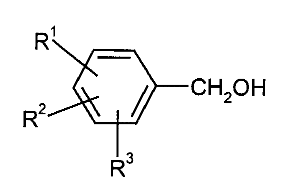 worin
R¹, R ² und R ³ die oben genannte Bedeutungen haben,
mit Ameisensäure in Gegenwart einer oder mehrerer Säuren zu Ameisensäurebenzylestern der Formel 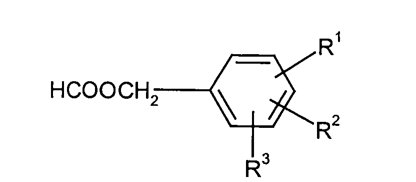 worin
R¹, R ² und R ³ die oben genannte Bedeutungen haben, umsetzt
und
diese Ameisensäurebenzylester in Gegenwart eines oder mehrerer Metalle oder Metalloxide der Elemente aus den Gruppen 3, 4, 5, 6, 7, 8, 9, 10, 11, 12, 13 und 14, bevorzugt Elemente der Gruppen 4, 5, 6, 7, 8, 9, 10, 11, 12, 13 und 14, insbesondere bevorzugt der Elemente der Gruppen 11 und 12, des Periodensystems nach IUPAC zu Benzylalkoholen fragmentiert.

Das erfindungsgemäße Verfahren lässt sich kostengünstig und unter milden Reaktionsbedingungen durchführen.

Die Reste R¹ bis R³ haben im allgemeinen die folgende Bedeutung:

Alkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen. Beispielsweise seien Methyl, Ethyl, Propyl, iso-Propyl, Butyl, iso-Butyl, Pentyl, iso-Pentyl, Hexyl und iso-Hexyl genannt. Bevorzugt sind Methyl und Ethyl.

Alkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen. Beispielsweise seien Methoxy, Ethoxy, Propoxy, iso-Propoxy, Butoxy, iso-Butoxy, Pentoxy, iso-Pentoxy, Hexoxy und iso-Hexoxy genannt. Bevorzugt sind Methoxy und Ethoxy.

Halogenalkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethyl, Fluormethyl, Trifluormethyl, Fluorethyl, Fluorpropyl und Hexafluorbutyl genannt. Bevorzugt sind Fluormethyl und Trifluormethyl.

Halogenalkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethoxy, Fluormethoxy, Trifluormethoxy, Fluorethoxy, Fluorpropoxy und Hexafluorbutoxy genannt. Bevorzugt sind Fluormethoxy und Trifluormethoxy.

Halogen bedeutet im allgemeinen Fluor, Chlor, Brom und Jod, bevorzugt Fluor und Chlor.

Bevorzugte Substituenten für R¹ bis R³ sind Wasserstoff, Methyl, Trifluormethyl, Methoxy, Fluor oder Chlor.

Für das erfindungsgemäße Verfahren können unsubstituierten oder substituierten Dibenzylether eingesetzt werden.

Besonders bevorzugt wird unsubstituierter Dibenzylether eingesetzt.

In das erfindungsgemäßen Verfahren können auch Dibenzylether oder Dibenzylether/Benzylalkoholgemische, wie sie beispielsweise bei der Herstellung von Benzylalkohol aus Benzylchlorid anfallen, eingesetzt werden. Der Gehalt an Dibenzylether in dem Gemisch kann 50 bis 100 Gew.-%, bevorzugt 60 bis 99 Gew.-%, besonders bevorzugt 70 bis 98 Gew.-%, sein.

Als Zwischenprodukte im Rahmen des erfindungsgemäßen Verfahren können beispielsweise die folgenden Ameisensäurebenzylestern hergestellt werden:

Ameisensäurebenzylester, Ameisensäure-(2-chlorbenzyl)ester, Ameisensäure-(3-chlorbenzyl)ester, Ameisensäure-(4-chlorbenzyl)ester, Ameisensäure-(2-fluor-benzyl)ester, Ameisensäure-(3-fluorbenzyl)ester, Ameisensäure-(4-fluorbenzyl)ester, Ameisensäure-(4-brombenzyl)ester, Ameisensäure-(2-methylbenzyl)ester, Ameisensäure-(4-methylbenzyl)ester, Ameisensäure-(4-trifluormethylbenzyl)ester, Ameisensäure-(2-fluor-4-chlorbenzyl)ester, Ameisensäure-(2,4-dichlorbenzyl)ester, Ameisensäure-(2,4-difluorbenzyl)ester, Ameisensäure-(4-nitrobenzyl)ester und Ameisensäure-(2-fluor-4-methoxybenzyl)ester.

Das erfindungsgemäße Verfahren wird vorzugsweise unter Entfernung des gebildeten Wassers durchgeführt. Geeignet ist die Entfernung des Wassers durch Destillation oder durch Durchleiten eines inerten Gases wie beispielsweise Stickstoff. Bevorzugt werden zur Entfernung des gebildeten Wassers wasserentziehende Mittel eingesetzt, beispielsweise Zeolithe, Aluminiumoxide oder Tonerden. Das gebildete Wasser kann auch dadurch entfernt werden, dass man die Umsetzung in Gegenwart eines Anhydrids als wasserentziehendes Mittel durchführt.

Im erfindungsgemäßen Verfahren werden vorzugsweise 0,1 bis 50 Äquivalente an Ameisensäure, bevorzugt 0,5 bis 30 Äquivalente, besonders bevorzugt 1 bis 20 Äquivalente, bezogen auf Dibenzylether, eingesetzt.

Die Herstellung der Ameisensäurebenzylester nach dem erfindungsgemäßen Verfahren wird in Gegenwart von Säuren durchgeführt.

Im erfindungsgemäßen Verfahren werden Säuren sowohl als homogene als auch als heterogene Katalysatoren eingesetzt. Es ist möglich eine oder mehrere Säuren im Gemisch einzusetzen. Bevorzugt wird die Umsetzung in Gegenwart einer Säure durchgeführt.

Die als Katalysator für das erfindungsgemäße Verfahren eingesetzten Säuren haben im allgemeinen einen pH Wert von 1 bis 6, bevorzugt von 1 bis 5, insbesondere bevorzugt von 1 bis 4.

Geeignete Katalysatoren für das erfindungsgemäße Verfahren sind anorganische Säuren wie beispielsweise Schwefelsäure, Chlorwasserstoff, Bromwasserstoff, Jodwasserstoff, Flusssäure, Perchlorsäure, Chlorsulfonsäure oder Phosphorsäure, organische Säuren wie beispielsweise Trifluoressigsäure, Methansulfonsäure, Ethansulfonsäure, Benzolsulfonsäure, 4-Toluolsulfonsäure oder Trifluormethansulfonsäure und Lewissäuren wie beispielsweise Bortrifluorid, Aluminiumchlorid, Aluminiumbromid, Aluminiumiodid, Zinkchlorid, Zinnchlorid, Titanchlorid oder Zirkonchlorid.

Bevorzugt sind Schwefelsäure, Trifluormethansulfonsäure, 4-Toluolsulfonsäure und Bortrifluorid, besonders bevorzugt Schwefelsäure, Trifluormethansulfonsäure und Bortrifluorid.

Die Säuren können sowohl als homogener als auch aufgebracht auf einem inerten Träger als heterogener Katalysator eingesetzt werden.

Für das erfindungsgemäßen Verfahren werden als heterogene saure Katalysatoren vorzugsweise saure Ionenaustauscher wie beispielsweise sulfonylierte Polymere wie sulfonylierte Polystyrole, sulfonylierte Styrol-Divinylbenzol-Copolymerisaten und sulfonylierte Phenolformaldehyd-Harzen eingesetzt.

Bevorzugt sind sulfonylierte Polystyrole und sulfonylierte Styrol-Divinylbenzol-Copolymerisate, besonders bevorzugt sulfonylierte Polystyrole.

Die Ionenaustauscher sind durch Umsetzung von Polystyrol oder Styrol-Divinylbenzol-Copolymerisaten mit Sulfonierungsmitteln wie Schwefelsäure oder Chlorsulfonsäure zugänglich.

Methoden zur Herstellung der Ionenaustauscher sind bekannt und beispielsweise beschrieben in Encyclopedia of Polymer Science and Technology, 1967, Band 7, S. 695 ff.

Geeignet sind handelsübliche Ionenaustauscher wie beispielsweise Produkte vertrieben unter den registrierten Handelsnamen Lewatit®, Amberlyst®, Amberlite®, Dowex®, Duolite®, Nafion®, Permutit®, Chempro® und Imac®.

Die Ionenaustauscher können in Kugelform vorliegen und Korngrößen von 0,3 bis 3,0 mm Durchmesser aufweisen. Sie können vom Geltyp oder makroporös sein. Ihre Totalkapazität an Säurefunktionen in wasserfeuchter Form mit einem Wassergehalt von ca. 75 bis 85 Gew.-% reicht von 0,7 bis 2,1 mval/ml Ionenaustauscher oder von 3,5 bis 5 mval/ml, bezogen auf 1 g Trockensubstanz an Ionenaustauscher.

Diese Ionenaustauscher werden gegebenenfalls durch Wärme, gegebenenfalls im Vakuum, gegebenenfalls durch Waschen mit hydrophilen organischen Flüssigkeiten wie beispielsweise die eingesetzte Carbonsäure oder das eingesetzte Carbonsäureanhydrid oder gegebenenfalls durch azeotrope Destillation mit organischen Flüssigkeiten wie Toluol, Xylol oder Methylenchlorid getrocknet.

Die genannten Ionenaustauscher werden bei Arbeiten mit suspendiertem Katalysatoren in Rührgefäßen in Mengen von 0,1 bis 100 Gew.-%, bevorzugt von 0,5 bis 90 Gew.-% und besonders bevorzugt von 1,0 bis 80 Gew.-%, bezogen auf Dibenzylether, verwendet.

Im Falle einer kontinuierliche Fahrweise im Gegen- oder Gleichstrom oder in der Rieselphase am Festbettkatalysator werden Katalysatorbelastungen von 0,05 g bis 5000 g Dibenzylether pro Liter aufgequollenem Ionenaustauscher pro Stunde, bevorzugt von 0,1 bis 4000 g / l.h und besonders bevorzugt von 1,0 bis 3000 g / l.h verwendet.

In einer besonderen Ausführungsform des erfindungsgemäßen Verfahren werden als Katalysatoren Heteropolysäuren (Polyoxymetallate) der allgemeinen Formel

Hₐ X_{b} M_{c} O_{d}

worin
- H: für Wasserstoff und/oder Metallkationen steht,
- X: für Phosphor, Silizium, Bor oder Germanium steht,
- M: für Wolfram, Molybden, Vanadium oder Chrom steht und
- a: für die Zahlen 3, 4, 5 oder 6 steht, mit der Maßgabe, dass die Elektro-neutralität der Heteropolysäuren oder deren Salze gegeben ist,
- b: für die Zahlen 1 oder 2 steht,
- c: für die Zahlen 12 oder 18 steht und
- d: für die Zahlen 40 oder 62 steht,
eingesetzt

Als Metallkatione seien Alkalimetalle wie Lithium, Natrium, Kalium, Rubidium, Caesium oder Mangan, Nickel, Kobalt, Kupfer oder Lanthan genannt.

Bevorzugt sind Heteropolysäuren des Keggin-Typs ( Chem. Rev. 98 (1998) S. 12 und 64) d.h. Verbindungen mit b = 1, c = 12 und d = 40, wobei X für Wasserstoff steht.

Bevorzugt sind Molybden-, Wolfram- und Vanadiumoxiden mit Phosphorsäure oder Kieselsäure, wie beispielsweise Wolframatophosphorsäure, Wolframatokieselsäure, Molybdatophosphorsäure, Molybdatokieselsäure, Vanadatophosphorsäure und Vanadatokieselsäure. Bevorzugt sind. Wolframatophosphorsäure, Wolframatokieselsäure, Molybdatophosphorsäure, Molybdatokieselsäure, Vanadatophosphorsäure und Vanadatokieselsäure, besonders bevorzugt sind Wolframatophosphorsäure, Wolframatokieselsäure, Molybdatophosphorsäure und Molybdatokieselsäure.

Methoden zur Herstellung sind gut bekannt und beispielsweise beschrieben in "Römpp Lexikon Chemie, 10. Auflage, Stuttgart/New York,1997, Band 3, S. 1741 und Chemical Reviews 98, (1998), S. 3-49, Catal. Rev.-Sci. Eng., 37, 311 bis 352 (1995).

Die Heteropolysäuren gegebenenfalls deren Hydrate, können sowohl als homogener als auch auf einem inerten Träger als heterogener Katalysator eingesetzt werden.

Weiterhin für das erfindungsgemäßen Verfahren geeignet sind sulfatierte Oxide (Supersäuren) der allgemeinen Formel (I)

SO₃ . MₑO_{f} . xH₂O (I)

wobei
- M: für Zirkon, Titan, Hafnium, Aluminium, Silicium, Niob oder Tantal steht und
- e: für 1 oder 2 steht, so dass die Elektroneutralität der Oxide oder deren Salzen gegeben ist,
- f: für 2, 3 oder 5 steht,
- x: für 0 bis 10 steht

Beispielsweise sind sulfatiertes CaO, MgO, ZrO₂, TiO₂, HfO₂, SnO₂, Al₂O₃, SiO₂, Al₂O₃. SiO₂, Nb₂O₅, Ta₂O₅, Fe₂O₃, LaSO₄ oder CaSO₄ zu nennen.
Bevorzugt sind CaO, MgO, ZrO₂, TiO₂, HfO₂, SnO₂, Al₂O₃, SiO₂, Al₂O₃. SiO₂, Nb₂O₅, Ta₂O₅, Fe₂O₃, LaSO₄ oder CaSO₄, besonders bevorzugt sind CaO, MgO, SnO₂, ZrO₂, TiO₂, HfO₂, Al₂O₃, SiO₂, Al₂O₃. SiO₂, Nb₂O₅ und Ta₂O₅.

Methoden zur Herstellung sind gut bekannt und beispielsweise beschrieben in Applied Catalysis A, 146, 1996, S. 3 bis 32.

Die Säuren, gegebenenfalls deren Hydrate, können auf einen inerten Träger aufgebracht, gegebenenfalls kalziniert, als heterogener Katalysator eingesetzt werden.

Die Katalysatoren können z.B. als Pulver oder Formkörper eingesetzt werden und nach der Umsetzung z.B. durch Filtration, Sedimentation oder Zentrifugieren abgetrennt werden.

Für den Fall der Anordnung als Festbett werden die Säuren vorzugsweise auf einem Träger aufgebracht und als Formkörper, Z.B. als Kugeln, Zylinder, Stäbchen, Hohlzylinder, Ringe usw. eingesetzt.

Als Trägermaterial geeignet sind Aktivkohle, Kieselgel, Aluminiumoxid, Alumosilikate wie Zeolithe oder Schichtsilikate, Tonerden, Titanoxide, Zirkonoxide, Hafniumoxide und Salze wie beispielsweise Calciumsulfat.

Diese heterogenisierte Säuren werden gegebenenfalls durch Wärme, gegebenenfalls im Vakuum, gegebebenfalls durch Waschen mit hydrophilen organischen Flüssigkeiten wie beispielsweise die eingesetzte Ameisensäure oder gegebenenfalls durch azeotrope Destillation mit organischen Flüssigkeiten wie Toluol, Xylol oder Methylenchlorid getrocknet.

Die geträgerten Säuren werden bei Arbeiten mit suspendiertem Katalysatoren in Rührgefäßen in Mengen von 0,1 bis 100 Gew.-%, bevorzugt von 0,5 bis 90 Gew.-% und besonders bevorzugt von 1,0 bis 80 Gew.-%, bezogen auf Dibenzylether, verwendet.

Im Falle einer kontinuierliche Fahrweise im Gegen- oder Gleichstrom oder in der Rieselphase am Festbettkatalysator werden Katalysatorbelastungen von 0,05 g bis 5000 g Dibenzylether pro Liter immobilisierten Säure pro Stunde, bevorzugt von 0,1 bis 4000 g / l.h und besonders bevorzugt von 1,0 bis 3000 g / l.h verwendet.

Vorzugsweise wird das erfindungsgemäße Verfahren unter intensiver Durchmischung der Reaktionspartner durchgeführt. Eine intensive Durchmischung kann auf verschiedene, dem Fachmann bekannte Weisen, etwa durch Rühren, Düsen, Strombrecher, statische Mischer, Pumpen, turbulente Strömungen in engen Röhren und durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 568-570, Verlag Chemie, Weinheim 1988 näher beschrieben.

Nach dem erfindungsgemäßen Verfahren erhält man Ameisensäurebenzylester in guten Ausbeuten bei hohem Umsatz und guter Selektivität. Das erfindungsgemäße Verfahren ist ohne hohen apparativen Aufwand einfach durchführbar.

Eine bevorzugte Ausführungsform des erfindungsgemäßen Verfahrens ist indem man in eine Mischung oder Suspension von Säure, gegebenenfalls aufgebracht auf einem Träger, und Ameisensäure Dibenzylether zugibt und nach Beendigung der Reaktion den Katalysator durch z.B. Filtration oder Zentrifugieren abtrennt.

Eine weitere bevorzugte Durchführungsform ist das Gleichstromverfahren, bei der Dibenzylether und Ameisensäure im Gleichstrom beispielsweise von oben her auf eine in einem Rohr angeordnete Katalysatorschüttung aufgebracht und unten am Fuß des Rohres Ameisensäurebenzylester abgezogen werden.

In einer weiteren bevorzugten Ausführungsform des erfindungsgemäßen Verfahrens wird dieses in der Rieselphase durchgeführt und der heterogene Katalysator, vorzugsweise eine geträgerte Supersäure, liegt als Festbettkatalysator vor. Bevorzugt befindet sich die Katalysatorschüttung in einem senkrecht stehenden Rohrreaktor, welcher vorzugsweise Zwischenböden zur besseren Verteilung des Flüssigkeitsstroms und zur besseren Benetzung der Katalysatorschüttung enthält.

Die Aufarbeitung kann sowohl im Falle des suspendierten Katalysators als auch bei Festbettverfahrensvariante so durchgeführt werden, dass ein mit Wasser nicht mischbares Lösungsmittel, vorzugsweise Toluol, zu den Reaktionsprodukten gegeben wird. Nach der Abtrennung der organischen Phase, welche den rohen Ameisensäurebenzylester enthält, kann diese beispielsweise durch Destillation weiter gereinigt werden.

Das erfindungsgemäße Verfahren kann diskontinuierlich, kontinuierlich oder teilkontinuierlich durchgeführt werden.

Die Temperatur, bei das erfmdungsgemäße Verfahren durchgeführt wird, beträgt vorzugsweise 15 bis 200, besonders bevorzugt 25 bis 190, ganz besonders bevorzugt 30 bis 180°C.

Bei der Durchführung der Reaktion oberhalb 115°C muss entsprechend dem Dampfdruck unter erhöhtem Druck gearbeitet werden. Der benötigte Überdruck ist dann wenigstens gleich dem Dampfdruck des Reaktionsgemisches. Es kann bis etwa 50 bar betragen, vorzugsweise bis 25 bar.

Gegebenenfalls kann das erfindungsgemäße Verfahren unter einem üblichen Schutzgas wie beispielsweise Stickstoff, Helium oder Argon, durchgeführt werden.

Die nach dem erfindungsgemäßen Verfahren bei der nachfolgenden Fragmentierung von Ameisensäurebenzylestern zu Benzylalkoholen eingesetzte Katalysatoren sind Metalle oder Metalloxide der Elemente aus den Gruppen 3, 4, 5, 6, 7, 8, 9, 10, 11, 12, 13 und 14, bevorzugt Elemente der Gruppen 4, 5, 6, 7, 8, 9, 10, 11, 12, 13 und 14, insbesondere bevorzugt der Elemente der Gruppen 11 und 12, des Periodensystems nach IUPAC eingesetzt werden.

Die Fragmentierung kann beispielsweise in Gegenwart von Zink, Kupfer, Eisen, Zinn, Palladium, Rhodium oder Platin., bevorzugt in Gegenwart von Zink, durchgeführt werden.

Die Katalysatoren werden in Mengen von 0,01 bis 20,0 Gew.-%, bevorzugt von 0,1 bis 15,0 Gew.-%, besonders bevorzugt von 0,2 bis 1,0 Gew.-%, bezogen auf eingesetzte Ameisensäurebenzylester, eingesetzt.

Die Fragmentierung kann beispielsweise bei Temperaturen von 50 bis 250°C, bevorzugt bei 70 bis 230°C, besonders bevorzugt bei 100 bis 220°C durchgeführt werden:

Das erfindungsgemäßen Verfahren kann durch die folgenden Reaktionsgleichungen erläutert werden:

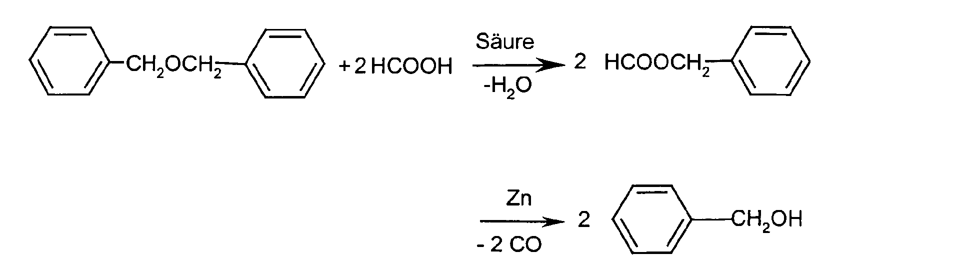

Nach dem erfindungsgemäßen Verfahren erhält man Ameisensäurebenzylester und Benzylalkohol in guten Ausbeuten bei hohem Umsatz und guter Selektivität. Das erfindungsgemäße Verfahren ist ohne hohen apparativen Aufwand einfach durchführbar.

### Beispiele

(Die Prozentangaben der nachfolgenden Beispiele beziehen sich auf das Gewicht)

### a) Herstellung Ameisensäurebenzylester aus Dibenzylether und Ameisensäure

### Beispiel 1

99,2 g (0,5 mol) Dibenzylether, 92,0 g (2,0 mol) Ameisensäure und 0,5 g konzentrierte Schwefelsäure wurden in einem Kolben mit Strombrecher und Flügelrührer unter kräftigem Rühren (250 U/min) und unter Stickstoff bei 90°C erhitzt. Nach 5 Stunden Reaktionszeit wurde rasch abgekühlt, die organische Phase nach Zugabe von Toluol und Wasser abgetrennt und gaschromatographisch analysiert. Das Reaktionsgemisch enthielt Benzylformiat und Dibenzylether im Verhältnis 57 zu 21.

### Beispiel 2

Beispiel 2 wurde analog zu Beispiel 1 mit 184,0 g (4,0 mol) Ameisensäure durchgeführt. Es wurden 0,5 g Trifluormethansulfonsäure eingesetzt. Die Reaktionszeit betrug 4 Stunden. Das Reaktionsgemisch enthielt Benzylformiat und Dibenzylether im Verhältnis 76 zu 3.

### Beispiel 3

Beispiel 3 wurde analog zu Beispiel 1 mit 184,0 g (4,0 mol) Ameisensäure und 3,0 g eines Ionenaustauschers Lewatit® SC 102 durchgeführt. Die Reaktionszeit betrug 7 Stunden. Das Reaktionsgemisch enthielt Benzylformiat und Dibenzylether im Verhältnis 53 zu 30.

### Beispiel 4

Beispiel 4 wurde analog zu Beispiel 1 mit 0,5 g Wolframatophosphorsäure (Firma Acros) durchgeführt. Die Reaktionszeit betrug 7 Stunden. Das Reaktionsgemisch enthielt Benzylformiat und Dibenzylether im Verhältnis 56 zu 23.

### Beispiel 5

Beispiel 2 wurde analog zu Beispiel 1 mit 184,0 g (4,0 mol) Ameisensäure und 3,0 g eines sulfatierten Tantaloxids (15 % SO₃ / 1 Ta₂O₅) durchgeführt. Die Reaktionszeit betrug 7 Stunden. Das Reaktionsgemisch enthielt Benzylformiat und Dibenzylether im Verhältnis 73 zu 6.

### Beispiel 6 (Aufarbeitung)

Beispiel 2 wurde wiederholt und nach 7 Stunden Laufzeit aufgearbeitet. Das Reaktionsgemisch enthielt Benzylformiat und Dibenzylether im Verhältnis 58 zu 17. Nach Neutralisierung mit Natriumbicarbonat, Extraktion mit Methylenchlorid, Trocknung über Natriumsulfat und destillativer Auftrennung der organischen Phase isoliert man bei 93 bis 99°C / 31 mbar 54,4 g (40 %) Benzylformiat mit einer Reinheit von 93,3 %, das noch 5 % Benzylalkohol enthält.

### Beispiel 6 (Aufarbeitung)

Beispiel 5 wurde nach 7 Stunden Laufzeit aufgearbeitet. Nach Neutralisierung mit Natriumbicarbonat, Extraktion mit Methylenchlorid, Trocknung über Natriumsulfat und destillativer Auftrennung der organischen Phase isoliert man bei 87 bis 91°C /23 mbar 69,6 g (51 %) Benzylformiat mit einer Reinheit von 97,2 %, im Rückstand verbleiben 8,7 g (8,7 %) Dibenzylether, dh. 56 % Benzylformiat bezogen auf umgesetztem Dibenzylether.

### b) Herstellung Benzylalkohol aus Ameisensäurebenzylester

### Beispiel 7

Eine Mischung aus 1,4 g (0,01 mol) Ameisensäurebenzylester und 0,2 g Zink-Pulver (Firma Acros) wurde bei 150°C unter Stickstoff erhitzt. Die Reaktionszeit betrug 8 Stunden. Das Reaktionsgemisch enthielt Benzylalkohol und Ameisensäurebenzylester im Verhältnis 11 zu 86.

### Beispiel 8

Beispiel 7 wurde wiederholt bei 175°C. Die Reaktionszeit betrug 8 Stunden. Das Reaktionsgemisch enthielt Benzylalkohol und Ameisensäurebenzylester im Verhältnis 28 zu 64.

### Beispiel 9

Beispiel 7 wurde wiederholt bei 190°C. Die Reaktionszeit betrug 5 Stunden. Das Reaktionsgemisch enthielt Benzylalkohol und Ameisensäurebenzylester im Verhältnis 47 zu 1.

## Patentansprüche

1. Verfahren zur Herstellung von Benzylalkoholen der Formel 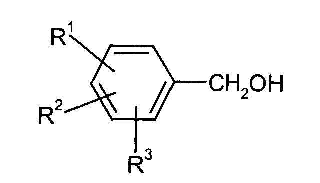 worin
R¹ bis R³ gleich oder verschieden sind und
für Wasserstoff, C₁-C₆-Alkyl, C₁-C₆-Alkoxy-, C₁-C₆-Halogenalkyl, C₁-C₆-Halogenalkoxy, CN, CO(C₁-C₆-Alkyl), NO₂ oder Halogen stehen,
**dadurch gekennzeichnet, dass** Dibenzylether der Formel 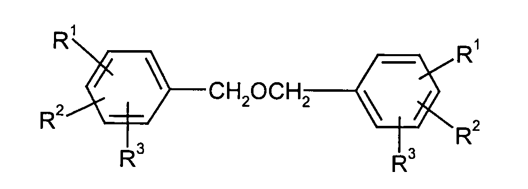 worin
R¹, R ² und R ³ die oben genannte Bedeutungen haben,
oder Gemische aus Dibenzylethern und Benzylalkoholen der Formel 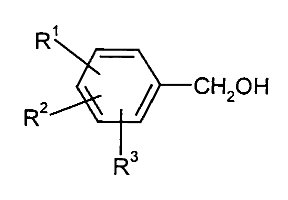 worin
R¹, R ² und R ³ die oben genannte Bedeutungen haben,
mit Ameisensäure in Gegenwart einer oder mehrerer Säuren zu Ameisensäurebenzylestern der Formel 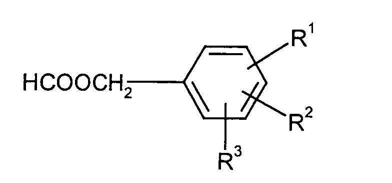 worin
R¹, R ² und R ³ die oben genannte Bedeutungen haben, umsetzt
und
diese Ameisensäurebenzylester in Gegenwart eines oder mehrerer Katalysators zu Benzylalkoholen fragmentiert.

2. Verfahren nach Anspruch 1, **dadurch gekennzeichnet, dass** die Säuren einen pH Wert von 1 bis 6 aufweisen.

3. Verfahren nach den Ansprüchen 1 und 2, **dadurch gekennzeichnet, dass** Säuren aus der Gruppe der anorganischen Säuren, organische Säuren, Brönstedsäuren oder der Lewissäuren eingesetzt werden.

4. Verfahren nach den Ansprüchen 1 bis 3, **dadurch gekennzeichnet, dass** als Säuren Schwefelsäure, Chlorwasserstoff, Bromwasserstoff, Jodwasserstoff, Flusssäure, Perchlorsäure, Chlorsulfonsäure, Phosphorsäure, Trifluoressigsäure, Methansulfonsäure, Ethansulfonsäure, Benzolsulfonsäure, 4-Toluolsulfonsäure, Trifluormethansulfonsäure, Bortrifluorid, Aluminiumchlorid, Aluminiumbromid, Aluminiumiodid, Zinkchlorid, Zinnchlorid, Titanchlorid oder Zirkonchlorid eingesetzt werden.

5. Verfahren nach den Ansprüchen 1 bis 4, **dadurch gekennzeichnet, dass** Säuren aus der Gruppe der sulfatierten Oxide (Supersäuren) der allgemeinen Formel
SO₃ . MₑO_{f} . xH₂O
wobei
M für Zirkon, Titan, Hafnium, Aluminium, Silicium, Niob oder Tantal steht und
e für 1 oder 2 steht,
f für 2, 3 oder 5 steht,
x für 0 bis 10 steht,
eingesetzt werden.

6. Verfahren nach den Ansprüchen 1 bis 3 und 5, **dadurch gekennzeichnet, dass** als sulfatierte Oxide (Supersäuren) sulfatiertes CaO, MgO, ZrO₂, TiO₂, HfO₂, SnO₂, Al₂O₃, SiO₂, Al₂O₃ · SiO₂, Nb₂O₅, Ta₂O₅ oder Fe₂O₃ eingesetzt werden.

7. Verfahren nach den Ansprüchen 1 bis 3, **dadurch gekennzeichnet, dass** Säuren aus der Gruppe der Ionenaustauscher eingesetzt werden.

8. Verfahren nach den Ansprüchen 1 bis 3 und 7, **dadurch gekennzeichnet, dass** als Ionenaustauscher sulfonylierte Polymere, sulfonylierte Styrol-Divinylbenzol-Copolymerisaten und sulfonylierte Phenolformaldehyd-Harzen eingesetzt werden.

9. Verfahren nach den Ansprüchen 1 bis 3, **dadurch gekennzeichnet, dass** als Säuren Heteropolysäuren (Polyoxymetallate) der allgemeinen Formel
Hₐ X_{b} M_{c} O_{d}
worin
H für Wasserstoff und/oder Metallkationen steht,
X für Phosphor, Silizium, Bor oder Germanium steht,
M für Wolfram, Molybden, Vanadium oder Chrom steht und
a für die Zahlen 3, 4, 5 oder 6 steht, mit der Maßgabe, dass die Elektro-neutralität der Heteropolysäuren oder deren Salze gegeben ist,
b für die Zahlen 1 oder 2 steht,
c für die Zahlen 12 oder 18 steht und
d für die Zahlen 40 oder 62 steht,
eingesetzt werden.

10. Verfahren nach den Ansprüchen 1 bis 9, **dadurch gekennzeichnet, dass** als Einsatzstoff Dibenzylether verwendet wird, welcher unsubstituiert oder durch einen oder mehrere Reste aus der Reihe C₁-C₆-Alkyl, C₁-C₆-Alkoxy-, CN, CO(C₁-C₆-Alkyl), NO₂ und Halogen substituiert ist.

11. Verfahren nach den Ansprüchen 1 bis 10, **dadurch gekennzeichnet, dass** 2 bis 50 mol-Äquivalente an Ameisensäure, bezogen auf 1 mol-Äquivalent Dibenzylether eingesetzt werden.

12. Verfahren nach den Ansprüchen 1 bis 11, **dadurch gekennzeichnet, dass** die Umsetzung in Gegenwart von wasserentziehenden Mitteln stattfindet.

13. Verfahren nach den Ansprüchen 1 bis 11, **dadurch gekennzeichnet, dass** die Umsetzung unter Entfernung von Wasser durch Destillation oder Durchleiten von Stickstoff stattfindet.

14. Verfahren nach den Ansprüchen 1 bis 13, **dadurch gekennzeichnet, dass** die Umsetzung im Temperaturbereich von 15 bis 200°C durchgeführt wird.

15. Verfahren nach den Ansprüchen 1 bis 14, **dadurch gekennzeichnet, dass** eine oder mehrere Säuren in einer Menge von 0,5 bis 100 Gew.-%, bezogen auf die Menge des Dibenzylethers, bei suspendiertem Katalysator bzw. mit Belastungen von 1,0 bis 3000g Dibenzylether pro Liter heterogenisierte Säure pro Stunde bei der Anordnung als Festbettkatalysator eingesetzt wird.

16. Verfahren nach den Ansprüchen 1 bis 15, **dadurch gekennzeichnet, dass** Katalysatoren bei der Fragmentierung von Ameisensäurebenzylestern zu Benzylalkoholen Metalle oder Metalloxide der Elemente aus den Gruppen 3, 4, 5, 6, 7, 8, 9, 10, 11, 12, 13 und 14 des Periodensystems nach IUPAC eingesetzt werden.

17. Verfahren nach den Ansprüchen 1 bis 16, **dadurch gekennzeichnet, dass** als Katalysatoren bei der Fragmentierung von Ameisensäurebenzylestern zu Benzylalkoholen Metalle oder Metalloxide der Elemente der Gruppen 4, 5, 6, 7, 8, 9, 10, 11, 12, 13 und 14 des Periodensystems nach IUPAC eingesetzt werden.
